# EUROPEAN PATENT APPLICATION

(11) **EP 2 179 793 A1**
(43) Date of publication of application: **28.04.2010**
(21) Application number: 08167109.1
(22) Date of filing: 21.10.2008
(51) Int. Cl.: B01J 27/199, C07C 51/215, B01J 23/652, B01J 37/04, C07C 51/25, C07C 253/24

(54) **Phosphorous-containing mixed oxide catalysts**

(71) Applicant: Süd-Chemie AG, 80333 München (DE)
(72) Inventor: Celaya Sanfiz, Almudena, Dr., 10777 Berlin (DE); Timpe, Olaf, Dr., 10405 Berlin (DE); Trunschke, Annette, Dr., 12489 Berlin (DE); Schlögl, Robert, Prof. Dr., 10779 Berlin (DE)
(74) Representative: Hoffmann, Klaus

(57) **Abstract**

The present invention relates to a tellurium-free mixed oxide catalyst comprising the elements (i) molybdenum (Mo), (ii) vanadium (V), (iii) at least one of niobium (Nb), tantalum (Ta) and tungsten (W) as well as (iv) phosphorous (P), and its use in the oxidation of hydrocarbons. Catalysts of this type show a surprisingly high selectivity in the oxidation of propane to acrylic acid.

## Description

The present invention relates to a catalyst material which is free of tellurium (Te) and antimony (Sb) and comprises as catalyst elements (i) molybdenum (Mo), (ii) vanadium (V), (iii) at least one of niobium (Nb), tantalum (Ta) and tungsten (W) as well as (iv) phosphorous (P), its manufacture and its use in the oxidation of (partially oxidized) hydrocarbons, in particular the direct oxidation of propane to acrylic acid.

### BACKGROUND OF THE INVENTION

Catalysts for alkane oxidation are usually complex multimetal oxides (MMO) containing molybdenum and vanadium oxide as essential constituents [1-3]. Particularly for selective oxidation of propane to acrylic acid, mixed oxides of molybdenum, vanadium, niobium, and tellurium have been identified in extensive compositional searches as the most promising catalysts [3], reaching yields of acrylic acid of about 50 %. Such mixed oxides are described for instance in [29] and [31]-[40]. Currently, acrylic acid is industrially produced in a two-step oxidation process starting from propene [4-5] over promoted bismuth molybdate and Mo-V mixed oxide catalysts achieving yields up to 86 %. Despite the lower price of propane compared to propene, the yield of acrylic acid in the direct oxidation of propane is still below the profitability threshold at actual commercial conditions. Thus, further improvement of catalysts for selective activation of alkanes is desired.

MoVTeNbOₓ catalysts mainly consist of two orthorhombic phases called "M1" and "M2" [6]. The structural and chemical complexity of M1 seems to cope with the challenging task of propane activation [7, 8], while M2 is regarded to be active and selective in oxidation of propene to acrylic acid [7, 9]. The M2 phase is richer in tellurium than M1. It was postulated that Te is an active element in propene conversion [8], but its beneficial effect found empirically may also be related to the generation of Te-free active sites. Specifically, tellurium seems to play a critical role with respect to phase formation. It was assumed that Te has structure-directing and structure-stabilizing effects regarding the M1 phase. However, Ueda and co-workers showed that a Mo-V oxide with a related orthorhombic structure may also be obtained by crystallization in inert atmosphere at 773 K in absence of tellurium [10], proposing that NH₄⁺ serves as structure-directing component [11]. A structure stabilizing role has been attributed to Te based on the observation that the precursor material of the ternary Mo-V-Te oxide treated at 873 K crystallizes in the desired orthorhombic structure, whereas the binary Mo-V system decomposes into other phases at such high temperatures [10-12]. Orthorhombic MoVOₓ and MoVTeOₓ systems show similar activity in the partial oxidation of propane to acrylic acid. However, the tellurium-containing catalyst is significantly more selective to acrylic acid [11-12]. Therefore, tellurium seems to play an essential role in view of the specific catalytic properties of these catalysts. However, the concentration of tellurium on the surface under operating conditions is poorly defined. Another disadvantage consists in the volatility of elemental tellurium at temperatures higher than 723 K that causes substantial Te loss during thermal activation of the catalyst and contamination of reactors and exhaust systems. The substitution of this element is, therefore, not only of interest for a deeper understanding of the role of tellurium in catalysis, but also for the prevention of tellurium contamination during catalyst preparation and operation. For similar reasons, the presence of the volatile element antimony (Sb) is considered undesired by the present inventors.

As to a discussion of M1 and M2 phases, their preparation and characterization reference can also be made to [20]-[23]. Recently, tellurium has been substituted by a number of metals in hydrothermally synthesized Mo-V-X ternary oxides with either X=Al, Fe, Cr, Ti [13], or X=Al, Ga, Bi, Sb, Te [14] and Mo-V-X-Nb quaternary oxides with (X=Te, Sb) [15]. The orthorhombic M1 structure was achieved only through substitution of Te by Sb in the ternary as well as in the quaternary system [14, 15]. Specifically, Ueda describes in [14] a Mo-Ni-Bi-O complex metal oxide catalyst suitable for the oxidation of propane to acrylic acid, albeit with an unsatisfactory selectivity of 31%. One further bismuth-containing mixed metal oxide catalyst of the type MO₆V₂Bi₁Oₓ, is disclosed in connection with the oxidation of ethane to acetic acid for which a selectivity of only 5.2% is given. The oxidation of propane to acrylic acid by means of MO₁₂W₂Bi₁Co_{5.5}Fe₃Si_{1.6}K_{0.08}Oₓ at a selectivity of 14% is known from [36].

Tellurium was also partly replaced by cesium in the M2 structure. The maximum tellurium substitution was 30 % [9]. With such a catalyst, improved activity for the ammoxidation of propene to acrylonitrile was observed. Tellurium-free catalyst materials which contain phosphorous and vanadium and/or molybdenum and their use in the oxidation of propane are also described in [25]-[28]. V₁P_{1.15}Te_{0.1-0.15}O has been reported in [24]. All these systems furnish relatively low selectivities (28%-32%) in the oxidation of propane to acrylic acid. A selectivity of 55% has been reported in [30] for a Te-Ni-Mo-O mixed metal oxide catalyst.

[41] (WO 2008/046843) discloses mixed oxide catalysts of the formula (Mo Biₐ B_{b} C_{c} D_{d} Eₑ F_{f} G_{g})Oₓ (I), wherein
B: at least one of nickel and/or cobalt
C: at least one of Fe, Ce, Mn, Cr, V,
D: at least one of W, P,
E: at least one of Li, K, Na, Rb, Cs, Mg, Ca, Ba, Sr,
F: at least one of Zn, Nb, Se, Te, Sm, Gd, La, Y, Pd, Pt, Ru, Ag, Au,
G: at least one of Si, A1, Ti, Zr,
   and a=0-1, b=0-1, c=0.001-1, d=0.01-2, e=0-1, f=0.01-1.5, g=0-800

Example 2 of this document relates to (MoCcr_{0.0286}Bi_{0.05})Oₓ and the remaining examples to tellurium-containing catalysts.

[42] (WO 2005/120702) purports to provide an improved hydrothermal preparation process for broadly defined multi metal oxide catalysts containing molybdenum and vanadium as essential elements. Example 5 of this document relates to the preparation of MO₁V_{0.25}Nb_{0.098}Bi_{0.042}Oₓ. The selectivities for the formation of acrylic acid achieved with these catalysts do not exceed 45 mol percent.

[43] (DE 10 2006 018886 A1) discloses the preparation of Mo₁V_{0.25}Nb_{0.12}Oₓ catalysts and their characterization without assessing their catalytic activity.

[44] (WO 2007/118870 A2) pertains to the same catalyst types and further catalysts such as Mo-V-Nb-Pb-O and Mo-W-V-Ti-Ta-O catalysts. Those examples relating to the oxidation of propane report the conversion degree of this starting material and state that acrylic acid was detected as reaction product.

[45] (EP 1 192 983 A1) relates to a catalyst comprising a promoted mixed metal oxide having the empirical formula MoₐV_{b}N_{c}X_{d}ZₑO_{f} wherein
N is at least one of Te, Sb, Sn, Ge and Bi,
X is at least one of Nb, Ta, Ti, A1, Zr, Cr, Mn, Fe, Ru, Co, Rh, Ni, Pt, B, Ga, As, Li, Na, K, Rb, Cs, Fr, Be, Mg, Ca, Sr, Ba, Hf, Pb, P, Pm, Eu, Gd, Dy, Ho, Er, Tm, Yb, Lu, La, Sc, Au, Ag, Pd, Ga, Pr, Re, Ir, Nd, Y, Sm, Tb, W, Ce, Cu and Zn, and Z is selected from In and Re;
and wherein, when a=1, b=0.01 to 1.0, c=0.01 to 1.0, d=0.01 to 1.0, e=0.001 to 1.0 and f is dependent on the oxidation state of the other elements. All concrete examples contain tellurium as catalyst element.

The subject-matter of [46] (WO 2005/094506) is a catalyst composition of the formula Mo₁VₐSb_{b}Nb_{c}M_{d}Oₓ wherein Mo is molybdenum, V is vanadium, Sb is antimony, Nb is niobium, M is gallium, bismuth, silver or gold, a is from 0.01 to 1, b is 0.01 to 1, c is 0.01 to 1, d is 0.01 to 1, and x is determined by the valence requirements of the other elements present.

According to further embodiments phosphorous may be present as one among many catalyst elements. The selectivities achieved for acrylic acid with concrete embodiments of this generic catalyst composition do not exceed 57.1 %.

[47] (WO 2008/013376) relates to tellurium-free Mo-Bi-Nb-based composite metal oxides and their use as catalyst in the production of (meth)acrylic acid. These catalysts may also contain W, Ta or V as one among many optional catalyst elements. The concrete examples given in this document conform to the formula Mo₁₂Bi_{1.2}Fe_{1.2}Co₅K_{0.05} or Mo₁₂Nb_{0.5}Bi_{1.2}Fe_{1.2}Co₅K_{0.05}. This document does not specify the reasons why these catalysts shall be tellurium-free.

In view of these results there still exists a demand for tellurium-free mixed oxide catalysts that oxidize propane to acrylic acid in high selectivities.

The possibility to improve the productivity of multi-metal oxide catalysts by adding diluents or carriers such as silica, alumina, titania, zirconia, and niobium oxide has also been studied [16-18]. MCM41 was used to prepare highly dispersed Mo-V-Te oxide catalysts for direct oxidation of propane to acrolein [19].

In view of the above, it is one object of the present invention to provide a catalyst material that shows a high selectivity in the oxidation of propane to acrylic acid and overcomes the problems associated with the use of tellurium and antimony in molybdenum-based mixed oxide catalysts of the aforementioned type.

### References

[1] M.M.Lin, Applied Catalysis A: General 207(2001), 1-2, 1.
[2] F. Borgmeier, A. Tenten, H. Hibst, E. K. J. Mueller, S. Unverricht, G. Cox, WO02/06199 (2002); BASF.
[3] T. Ushikubo, H. Nakamura, Y. Koyasu, S. Wajiki, US Patent 5 380 933 (1995); Mitsubishi Kasei Corporation.
[4] M. Tanimoto, H. Yunoki, H. Hironaka, N. Kimura, US 6 632 965 B1 (2003); Nippon Shokubai.
[5] F. Borgmeier, J. Petzoldt, H. Hibst, A. Tenten, WO 2002/083615 A1 (2002); BASF.
[6] T. Ushikubo, K. Oshima, A. Kayou, M. Hatano, Studies in Surface Science and Catalysis 112 (1997), 473.
[7] M. Baca, A. Pigamo, J. L. Dubois, J. M. M. Millet, Topics in Catalysis 23 (2003), 39.
[8] W. Ueda, D. Vitry, T. Katou, Catalysis Today 96 (2004), 235.
[9] J. Holmberg, S. Hansen, R. K. Grasselli, A. Andersson, Topics in Catalysis 38 (2006), 1-3, 17.
[10] T. Katou, D. Vitry, W. Ueda, Chemical Letters 32 (2003), 1028.
[11] W. Ueda, D. Vitry, T. Kato, N. Watanabe, Y. Endo, Research on Chemical Intermediates 32 (2006), 3-4, 217.
[12] W. Ueda, D. Vitry, T. Katou, Catalysis Today 99 (2005), 43.
[13] W. Ueda, N. F. Chen, K. Oshihara, Chemical Communications (1999), 517.
[14] W. Ueda, K. Oshihara, Applied Catalysis A: General 200 (2000), 135.
[15] N. Watanabe, W. Ueda, Industrial & Engineering Chemistry Research 45 (2006), 607.
[16] J. Holmberg, R. Häggblad, A. Andersson, Journal of Catalysis 243 (2006), 2, 350.
[17] J. B. Wagner, O. Timpe, F. A. Hamid, A. Trunschke, U. Wild, D.S. Su, R.K. Widi, S.B.A. Hamid, R. Schlögl, Topics in Catalysis 38 (2006), 51.
[18] B. Solsona, M. I. Vázquez, F. Ivars, A. Dejoz, P. Concepción, J. M. López-Nieto, Journal of Catalysis 252 (2007), 2, 271.
[19] L. Chen, J. Liang, H. Lin, W. Weng, H. Wang, J.C.Védrine, Applied Catalysis A: General 293 (2005), 49.
[20] P. DeSanto Jr., D. J. Buttrey, R. K. GrasselliI, C. G. Lugmair, A. F. Volpe Jr., B. H. Toby, T. Vogt, Zeitschrift für Kristallographie 219 (2004), 152.
[21] H. Hibst, F. Rosowski, G. Cox, Catalysis Today 117 (2006), 234.
[22] Preparation of phase-pure M1 MoVTeNb oxide catalysts by hydrothermal synthesis - Influence of reaction parameters on structure and morphology. A. Celaya Sanfiz, T. W. Hansen, E. Rödel, O. Timpe, A. Trunschke, R. Schlögl, (accepted for publication in Topics in Catalysis*)*
[23] R. K. Grasselli, Catalysis Today 99 (2005), 23.
[24] M. Ai, Journal of Catalysis 101 (1986), 389.
[25] Z. Wang, W. Wei, G. Liu, G. Mao, D. Kuang, Acta Petroleum Sinica 14 (1998), 21.
[26] W. Ueda, Y. Suzuki, Chemical Letters (1995), 7, 541.
[27] W. Li, K. Oshihara, W. Ueda, Applied Catalysis A: General 182 (1999), 357.
[28] N. Mizuno, M. Tateishi, M. Iwamoto, Applied Catalysis A: General 128 (1995), L165.
[29] D. Vitry, Y. Moriwaka, J. L. Dubois, W. Ueda, Applied Catalysis A: General 251 (2003), 411.
[30] N. Fujikawa, K. Wakui, K. Tomita, N. Ooue. Catalysis Today 71 (2001), 83.
[31] P. Botella, J. M. López-Nieto, B. Solsona, A. Mifsud, F. Marquez, Journal of Catalysis 209 (2002), 445.
[32] M. Baca, M. Aouine, J. L. Dubois, J. M. M. Millet, Journal of Catalysis 233 (2005), 234.
[33] J. M. Oliver, J. M. López-Nieto, P. Botella, Catalysis Today 96 (2004), 241.
[34] E. Balcells, F. Borgmeier, I. Grisstede, H.-G. Lintz, F.Rosowski, Applied Catalysis A: General 266 (2004), 211.
[35] P. Botella, P. Concepción, J. M. López-Nieto, Y. Moreno, Catalysis Today 99 (2005), 51.
[36] H. Jachow, A. Tenten, S. Unverricht, H. Arnold, US 6 541 664 (2003); BASF.
[37] H. Hibst, F. Borgmeier, F. Rosowski, K. J. Müller-Engel, DE 10 2004 027 999 A1 (2005); BASF.
[38] F. Borgmeier, F. Rosowski, H. Martan, K. J. Müller-Engel DE 10 344 265 A1 (2004); BASF.
[39] M. Abon, K. E. Bere, A. Tuel, P. Delichere, Journal of catalysis 156 (1995), 28.
[40] J. Holmberg, R. K. Grasselli, A. Andersson, Applied Catalysis A: General 270 (2004), 121.
[41] (WO 2008/046843)
[42] (WO 2005/120702)
[43] (DE 10 2006 018886 A1)
[44] (WO 2007/118870 A2)
[45] (EP 1 192 983 A1)
[46] (WO 2005/094506)
[47] (WO 2008/013376)

### SUMMARY OF THE PRESENT INVENTION

The present invention relates to
I. a method for preparing a mixed oxide catalyst material which is free of tellurium (Te) and antimony (Sb) and comprises (i) molybdenum (Mo), (ii) vanadium (V), (iii) at least one of niobium (Nb), tantalum (Ta) and tungsten (W), and (iv) phosphorous (P), said method comprising the steps of
   (A) subjecting a catalyst precursor mixture, which comprises the elements (i) to (iv) and may optionally contain a diluent, to a calcination treatment in an oxygen-containing atmosphere at a temperature of 150 to 400°C, preferably 200 to 350°C to prepare a calcined catalyst material, and
   (B) subjecting the catalyst material obtained in step (A) to an activation treatment in an inert gas atmosphere at a temperature of 350 to 700°C, preferably 580 to 670°C;
II. said mixed oxide catalyst material and a catalyst containing the same; and
III. its use as catalyst in oxidation reactions of hydrocarbons or partially oxidized hydrocarbons and the corresponding method.

### DETAILED DESCRIPTION OF THE INVENTION

In the following the use of "comprising" also includes more restricting embodiments where "comprising" is replaced by "essentially consisting of" or "consisting of". Further, hereinafter the explicit disclosure of a broader quantitative range of values (e.g. a-d) together with an included (preferred) narrower range (e.g. b-c) also discloses the two possible part-ranges lying within the broader range on either side of the narrower range. Hence, within this broader quantitative range of values (e.g. a-d), further embodiments are characterized by all ranges (e.g. a-b, a-c, b-d or c-d) which can be formed by combining any of the upper limit or the lower limit of this explicitly defined broader quantitative range with the lower limit or the upper limit of any explicitly defined and included (preferred) narrower range.

### I. Catalyst preparation method

According to the **first step (A)** of the claimed preparation method, a *"catalyst precursor mixture"* is subjected to a **calcination treatment** in an oxygen-containing atmosphere (air or a synthetic oxygen-containing atmosphere) at a temperature of 150 to 400°C to prepare a calcined catalyst precursor. The catalyst precursor mixture comprises (i) molybdenum (Mo), (ii) vanadium (V), (iii) at least one of niobium (Nb), tantalum (Ta) and tungsten (W) and (iv) phosphorous (P) and optionally other catalyst metal elements such as Z defined below (in the following these catalyst elements are abbreviated as *"catalyst metals"* although, strictly speaking, phosphorous (P) is not a metal). In this precursor mixture and in the final catalyst material the molar ratio P/Mo is preferably at least 0.05/1, more preferably at least 0.1/1, e.g. 0.05/1 to 0.5/1.

The catalyst precursor mixture can be obtained according to any commonly known process.

As *"starting material"* for the catalyst precursor mixture we understand chemical compounds that comprise catalyst metals and can be converted to metal oxides in the calcination treatment or already contain such oxides (the expression "can be converted" does not exclude that this conversion already takes place at least in part at an earlier point in time, for instance during drying at higher temperature). The starting material is preferably selected from oxides of the catalyst metals, salts of the catalyst metals, metal-containing acids, in particular oxyacids, metalorganic compounds and organic metal complexes. More preferably it is selected from metal oxides, inorganic or organic salts and (oxy)acids. It is possible to prepare the catalyst precursor mixture by combining as many metal-containing starting materials as metals are to be included in the final catalyst or by adding at least one starting material that contains more than one of these metals.

According to one preferred embodiment, the catalyst precursor mixture to be calcined is prepared under so-called ***"hydrothermal"*** conditions that is in the presence of water under elevated temperature and pressure. The hydrothermal preparation comprises the steps of
(a) dissolving or dispersing starting materials containing at least the catalyst metals (i) to (iv), if required under heating,
(b) combining, optionally under heating, the resulting solution(s) and/or dispersion(s) to an aqueous mixture containing the catalyst metals in the desired ratio for the final catalyst material,
(c) heating the aqueous mixture at elevated pressure (i.e. above atmospheric pressure),
(d) collecting and drying the solid material formed in step (c).

Suitable starting materials (metal sources) for Mo, V and Nb oxides are for instance those described in US 5,380,933 (col. 3, line 27 to 57) and/or US 6,710,207 (col. 8, lines 12 to 30), and include organic and inorganic salts and acids (normally oxyacids) of the desired metal elements. The salts are selected in a manner that after calcining only metal elements and oxygen remain in the calcined catalyst precursor since all other constituents are volatile or rendered volatile by decomposition or oxidation. For this reason, the use of ammonium salts of the metal element (or the corresponding oxyacid), organic salts such as oxalates, alkoxides or acetylacetonates, organic metal complexes, metalorganic compounds or volatile inorganic salts such as nitrates is preferred. Moreover, the selected salts and acids are preferably soluble or at least dispersible in the selected solvent such as water. According to one embodiment, the water-solubility of the starting salt or acid is at least 0.1 mol metal per 11 of water at 373K (100°C). Suitable starting salts and acids include for instance ammonium para- or heptamolybdate, molybdenum oxalate, molybdophosphoric acid, bismuth nitrate, ammonium metavanadate, vanadium oxalate, vanadyl sulfate (VOSO₄), ammonium niobium oxalate, ammonium para- or heptatungstate, tungsten oxalate, tungstophosphoric acid, and ammonium tantalum oxalate.

According to one embodiment, the first step involves the preparation of a solution or dispersion containing Mo and the element X (P, optionally replaced in part by Bi). For instance, molybdophosphoric acid is prepared by dissolving MoO₃ in phosphoric acid, or bismuth nitrate is added to an aqueous dispersion (slurry) of MoO₃ or *vice* versa. In the second step, a solution or dispersion of the V source (e.g. an aqueous V₂O₅ dispersion or an aqueous ammonium metavanadate solution) is added to the solution or dispersion prepared in the first step, followed by the addition of the solution of a Nb source (e.g. an aqueous solution of ammonium niobium oxalate). Similar or identical techniques can be used if Nb is partially or fully replaced by Ta and/or W.

Step (c) is preferably conducted at a temperature of above 100°C up to 250°C, for instance 120 to 200°C, preferably 130°C to 180°C at a pressure of more than 0.2 MPa (2 bar). There is no specific upper pressure limit (it is for instance possible to work with pressure up to 9MPa). For practical reasons, normally pressures of 0.3 to 2MPa, such as 0.4 to 1 MPa are used. Typically, the reaction is conducted in a closed reactor, such as an autoclave to make use of the autogeneous pressure generated at higher temperatures by the existing water. Furthermore, the reaction time at the maximum temperature and pressure can be varied considerably and may for instance range from 5 min to 144 hours. According to further embodiments, the reaction time ranges from 10 min to 130 hours, or 20 min to 48 hours or 30 min to 24 hours. It is not considered necessary to work under oxygen-free conditions but it is preferred to replace air existing in the reactor by an inert gas. The reaction is preferably conducted in autoclaves made from a suitable inert material (e.g. Hastelloy C276) or having a suitable inner lining (e.g. Teflan).

It is preferred that the drying process does not eliminate any remaining moisture in the material to be calcined. Typically, the drying process (e.g. spray-drying) is terminated if the particles to be calcined do no longer agglomerate. Excessive drying is to be avoided in order to preserve residual moisture, which is believed to be beneficial in transport phenomena. Excessive drying occurs if the dried particles start to dust.

According to one further embodiment, the catalyst precursor mixture is prepared in a wet mixing method which preferably comprises the steps of
(a) preparing a solution or dispersion of starting materials (e.g. salts and/or acids) comprising the elements (i) to (iv), and optionally at least one further metal to be added to the catalyst such as element Z defined below, in a solvent,
(b) optionally precipitating dissolved starting materials of these metals,
(c) removing the solvent from the solution, dispersion or optionally the precipitate to form a solid mixture, and
(d) drying said solid mixture to form a catalyst precursor mixture.

Steps (b), (c) and (d) can be performed subsequently or simultaneously, as in one embodiment of the present invention (spray-drying).

To prepare solutions or dispersions that contain the catalyst metals, suitable starting materials are typically dissolved or dispersed in predetermined ratios in an appropriate solvent, if required under heating, and combined.

The subsequent isolation of a dry catalyst precursor mixture can be effected in a usual manner by e.g. filtration, evaporation to dryness (e.g. rotary evaporation), spray drying, freeze drying, drying at ambient air and/or vacuum drying.

Solvents that can be used in the preparation of the catalyst precursor mixture are not specifically limited, and preferred solvents include water, alcohols, preferably methanol, ethanol, propanol and butanol, diols, such as ethylene glycol or propylene glycol, and other polar solvents. Water is more preferred. Further, any suitable mixture of the above solvents can be used.

As to the drying process, reference is made to the previous comments. Regarding the choice of suitable starting materials, further embodiments and preparation conditions reference is also made to the previous description as long as the same does not contradict the wet mixing method.

The calcination and activation of a catalyst precursor mixture that was obtained as described above leads to the claimed catalyst material which shows an excellent selectivity in the direct oxidation of propane to acrylic acid.

The molar ratio of starting materials in terms of metal components is chosen in accordance with the desired **final composition** of the catalyst. The final composition is a mixed oxide which comprises oxides of the four elements (i) to (iv), and optionally oxides of other metal elements, as long as these do not adversely affect the function of the resulting material as a catalyst in the oxidation reactions referred to herein.

Preferably, the final average catalyst composition lies within the ranges defined by general formula (I):

Mo₁VₐX_{b}Q_{c}Z_{d}Oₑ (I)

wherein
X is P, which may be replaced in part by Bi, for instance up to a molar ratio Bi/P of 1/1,
Q is at least one of Nb, Ta and W (which includes the combined use of Nb and Ta, Nb and W, and Ta and W, as well as the use of all three elements),
a = 0.15-0.50, b = 0.02-0.45, in particular 0.05-0.40, c = 0.05-0.45, d ≤ 0.05 and e is the molar number of oxygen binding to the metal atoms present in this mixed oxide which follows from the relative amount and valence of the metals elements, and Z is at least one element selected from Na, K, Si, Ru, Mn, Sc, Ti, Cr, Fe, Co, Ni, Cu, Zn, Ga, Y, Zr, Rh, Pd, In, Ce, Pr, Nd, Sm, Tb, Re, Ir, Pt, Au, and Pb.

"Average" composition means the composition as can be determined with techniques such as XRF particularly suitable for analyzing the bulk elemental composition.

According to one embodiment of the present invention d is 0 in formula (I). According to one further embodiment, the catalyst of formula (I) is specifically free of iron.

If in the compound of the formula (I) the at least one optional element Z is present (i.e. d > 0), it is preferably at least one element selected from Na, K, Si, Ru, Mn, Cr, Fe, Co, Ni, Zr, Rh, Pd, In, Ce, and Pt. More preferred are compounds of formula (I), wherein Z, if present, is at least one element selected from Cr and Ni. Another preferred embodiment relates to the use of Ru, Cu, Rh, Re and/or Mn as Z element, Ru, Mn and Cu, in particular Ru and Mn being particularly preferred. If element Z is present, the lower limit of d is for instance 0.0001, 0.0005 or 0.001.

In particular noble metals are effective at very low levels such as several 100 ppm based on the Mo content. According to one embodiment, the above mixed oxide catalyst material of formula (I) contains at least one noble metal as element Z. It is preferred that in formula (I) Z is at least one of the noble metals Rh, Pd, Ir, Pt and Au, a = 0.25-0.35, b = 0.20-0.30, c = 0.10-0.15, and d = 0.0001 to 0.05, for instance 0.0003 to 0.005.

According to one more preferred embodiment, the final average catalyst composition of formula (I) lies within the following ranges:
a = 0.20-0.40, preferably 0.25-0.35, b = 0.15-0.35, preferably 0.20-0.30, c = 0.07-0.18, preferably 0.10-0.15, and d and e are as defined above.

When selecting the ratio of starting materials it should be considered that during the calcination step and the activation treatment the molar ratio of catalyst metals may change to a certain extent. MoO₃ which may have undesirably formed in minor amounts is for instance volatile at temperatures above 700°C. Phosphorous levels may also be reduced slightly.

In one embodiment the catalyst precursor mixture to be calcined, and thus also the final catalyst material, can include a **solid diluent.** As diluent, any inert material, that can withstand the conditions of the calcination and the activation treatment and does not unfavorably interact with the mixed oxide catalyst material such that the catalytic activity thereof would be impaired.

The presence of a solid diluent may be beneficial for various reasons. First of all, preferred diluents are characterized by a higher thermal conductivity than the catalytically active mixed oxide material. This ensures a better heat transport management and prevents the formation of hot spots during the use of the catalyst, which could lead to undesired side reactions or lower the catalyst life. Further, the diluent functions as a separating agent for the catalytically active material and counteracts any sintering processes, which may occur between the grains of catalyst material. Further, the diluent may also improve the surface properties of the catalyst. The term "diluent" does not exclude that the material used also acts as promoter.

Suitable diluents may be selected for instance from metal oxides or carbides such as titania (TiO₂), carbon, chromium oxide (Cr₂O₃), alumina, zirconium oxide (zirconia), cerium oxide (CeO₂), SiC and silica.

According to one embodiment, the diluent is treated with a solution containing at least one catalyst metal defined in formula (I) prior to its admixture to the catalyst precursor material or a starting material thereof.

According to one further embodiment, the optionally pretreated and dried diluent is subjected to the same calcination procedure, as described for the catalyst precursor material, before it is combined with the catalyst starting material. Thus, the optionally pretreated diluent preferably undergoes this calcination twice, once prior to mixing with catalyst starting material and a second time together with this catalyst precursor material.

Preferably, the weight ratio of the diluent to the mixed oxide catalyst material is in the order of 1.5:1 to 0.5:1.

The diluent can be added at any time prior to the calcination procedure, i.e. it can be mixed with the catalyst metal-comprising starting materials in a dry or a wet state or, if the catalyst precursor mixture is prepared using a solvent, it can be added to the solvent to precipitate the catalyst starting materials on the diluent in the process of preparing the catalyst precursor mixture.

For the catalyst precursor mixture any of the aforementioned preparation procedures may be chosen. In the solid material resulting from this preparation procedure (=catalyst precursor mixture) a diluent may be present. The resulting solid material, which may contain a diluent, may also be coated onto a carrier to give a catalyst precursor mixture (please see item II. below).

Any of these catalyst precursor mixtures is then subjected to a ***calcination in an oxygen-containing atmosphere (step A)*** at a temperature of 150-400°C, preferably 200-350°C, more preferably 250-300°C. This procedure is intended to remove volatile constituents and convert catalyst elements to their oxides, if necessary.

For the sake of convenience, this calcination reaction is usually conducted at atmospheric pressure. In principle it is, however, also possible to conduct this step under slightly elevated or slightly reduced pressure, for instance within the range of atmospheric pressure ± 50% or ± 20%. As oxygen-containing atmosphere, air or a synthetic oxygen-containing atmosphere can be used. Depending on the other process conditions, oxygen is normally not employed in contents of more than 50 vol.-%. Suitable oxygen volume ratios are for instance 1 to 40 vol.%, 5 to 35 vol.-% or 10 to 30 vol.-%. The remainder is nitrogen as in air or any other inert gas such as Ar or He.

In principle, any suitable reactor can be used for conducting the calcination. Especially if organic metal salts are used as starting material, it is preferred to calcine in a rotating oven which seems to enhance the complete removal of organic constituents.

The catalyst precursor mixture is gradually heated from the starting temperature (usually room temperature, that is 20°C) to the final calcination temperature, preferably at a rate of 1 to 20 K/min, in particular 5 to 15 K/min. Calcination is then conducted at the final temperature of 150 to 400°C, preferably 200-350°C, more preferably 250-300°C, preferably over a time period of 20 min to 20 hrs, for instance 30 min to 10 hrs, 1 hr to 5 hrs or 1 hr to 3 hrs.

During calcination the oxygen-containing atmosphere is preferably moved continuously relative to the catalyst precursor mixture. The expression "moved" relates to embodiments according to which fresh oxygen-containing atmosphere is supplied to replenish possibly consumed and therefore oxygen-depleted atmosphere. Suitable supply rates are for instance in the order of 10 to 100 ml/min, in particular 30 to 70 ml/min, based on a weight of catalyst precursor mixture of 1-50 g.

According to one preferred embodiment, normally after an intermediate cooling step, a second thermal treatment (also referred to as ***"activation treatment"* or step (B)**) follows the calcination treatment to further enhance desired properties such as the crystallinity and oxidation state. The activation treatment proceeds in an inert atmosphere, preferably in nitrogen gas or argon gas, at a temperature of 350-700°C, preferably 550-680°C, even more preferably 580-670°C, in particular 590 to 670°C. During this activation treatment it is particularly important to work in the absence of oxygen since the mixed oxide obtained after the calcination treatment is normally in a not fully oxidized state (as it contains at least in part molybdenum (Mo) or tungsten (W) in an oxidation state of less than +VI and at least in part vanadium (V) in an oxidation state of less than +V) and complete oxidation to MoO₃ or WO₃, or V₂O₅, respectively, is preferably to be avoided.

For the sake of convenience, this activation treatment is usually conducted at atmospheric pressure. In principle it is, however, also possible to conduct the treatment under slightly elevated or slightly reduced pressure, for instance within the range of atmospheric pressure ± 50% or ± 20%. The treatment time in this step is also not specifically limited, and is preferably 0.5-30 h, more preferably 1-20 h and in particular 1-10 h.

The catalyst precursor mixture is gradually heated from the starting temperature (usually room temperature that is 20°C) to the final activation temperature, preferably at a rate of 0.5 to 30 K/min, for instance 3 to 20 K/min, or 5 to 15 K/min.

### II. Metal oxide catalyst and material

The present invention also provides a new and superior mixed oxide catalyst material which shows excellent selectivity in the oxidation of hydrocarbons, in particular in the direct oxidation of propane to acrylic acid.

The term *"mixed oxide* is not be understood as mixture of the respective four or more mono metal oxides of the elements (i) to (iv) and element Z but as oxide phase that comprises at least one oxide having a defined stoichiometry and comprising two, tree or four of the essential catalyst elements (A-V-X-Q). The claimed catalyst material furnishes in certain embodiments amorphous mixed oxide phases (as determined under the XRD measuring conditions described in the experimental section).

Specifically, it has been found that the catalyst material of the invention preferably shows selectivity for acrylic acid formation in the direct oxidation of propane of at least 70%, in particular at least 75%, at least 80%, at least 85%, at least 90% and at least 95%, with increasing preference, under the oxidation conditions specified in example 4 with respect to the catalyst material of example 1.

The catalyst material of the invention can be used as **catalyst** as obtained after the activation treatment, that is as powder. According to one embodiment, catalyst particles of a definite size are formed from the obtained powdery catalyst material (which may also contain a diluent).

There is no specific limitation regarding the size of the catalyst particles to be used. The following structural features are however preferred.

The macroscopic size (average longest diameter) of the individual catalyst particles preferably ranges from 0,5 to 10mm. Catalyst particles of this size can be obtained by processes known in the art, for instance by pressing a dried catalyst starting material, newly crushing the pressed material and carrying out size-selecting steps such as sieving, before conducting the calcination step. Alternatively, the already calcined material is pressed, newly crushed and subjected to size-selecting steps such as sieving. Instead of pressing, an extrudate may be formed. Pressing and extrusion may be equally conducted with the activated catalyst material.

According to one further embodiment, the final catalyst material is coated onto a carrier according to techniques known in the art. This coating of a carrier with the respective catalyst (precursor) materials can be equally effected at an earlier stage, for instance prior to the calcination treatment or prior to the activation treatment.

The **carrier,** which is preferably inert, can have any shape and surface structure. However, regularly shaped, mechanically stable bodies such as spheres, rings, tube sections, half-rings, saddles, spirals or honeycomb carrier bodies or carrier bodies provided with channels such as, for example, fibre mats or ceramic foams are preferred. The size and shape of the carrier bodies is determined, for example, by the dimensions, primarily the internal diameter of the reaction tubes if the catalyst is used in tube or tube-bundle reactors. The diameter of the carrier body should then be between 1/2 and 1/10 of the internal diameter of the reactor. In the case of fluidised bed reactors, the carrier dimensions are determined, for example, by the fluid dynamics in the reactor. Suitable materials are, for example, steatite, duranite, stoneware, porcelain, silicon dioxide, silicates, aluminium oxide, aluminates, silicon carbide or mixtures of these substances. Tube sections, rings or spheres made of ceramic, silicon carbide or carbon are preferably used.

The proportion of the layer of catalyst material applied to the carrier is preferably 1 to 30% by weight, particularly preferred 2 to 20% by weight based on the total mass of the catalyst material (exclusive optionally present diluent). The thickness of the layer is preferably 5 to 300 µm, particularly preferred 5 to 10 µm.

In one embodiment, the selected carrier is coated with an aqueous slurry of the starting materials for the catalyst precursor material, or a suspension thereof in an organic solvent such as for example toluene, followed by the removal of water or organic solvent and the process steps described in item I (calcination, activation). If the carrier is coated at a later stage the same technique can be employed under use of an optional heat treatment after the removal of water or organic solvent to fix the catalyst material on the carrier.

### III. Use/method for oxidizing a hydrocarbon

One further aspect of the present invention relates to a method for oxidizing a (optionally partially oxidized) hydrocarbon in the presence of oxygen and a catalyst as defined above.

According to one preferred embodiment, this method comprises subjecting a hydrocarbon, in particular an alkane to a vapour phase catalytic oxidation reaction to produce an unsaturated carboxylic acid. This embodiment is preferably applied to C1 to C5 alkanes. For example, when propane or isobutane is used as starting alkane, acrylic acid or methacrylic acid will be obtained, respectively, in excellent selectivities.

In this embodiment the catalyst of the invention can be used under conventional conditions to convert hydrocarbons to unsaturated carboxylic acids. The reaction is preferably conducted in fixed bed reactors, for instance tubular fixed bed reactors. For reasons of convenience, atmospheric pressure can be used whilst the reaction proceeds similarly under lower or higher pressures. Preferably, an inert gas (e.g. nitrogen) and/or steam are admixed to the hydrocarbon (e.g. propane) and oxygen. A standard feed composition is for instance 0.5-5 vol.-% alkane (e.g. propane), 2-20 vol.-% oxygen and 10-65 vol.-% steam, the balance being inert gas such as nitrogen; preferably 1.5-3.5 vol.-% alkane (e.g. propane), 5-20 vol.-% oxygen and 20-50 vol.-% steam, the balance being inert gas such as nitrogen. Of course, propane / oxygen ratios that tend to be explosive should be avoided. Preferred reaction temperatures range from 350 to 450°C. The molar amount of steam (H₂O) based on the total molar amount of hydrocarbon, O₂, inert gas (e.g. N₂) and steam (H₂O) can be varied considerably with the catalyst of the invention. Suitable results are achieved with molar amounts of preferably 5-65%, for instance 10-50%.

According to one further embodiment, a saturated or unsaturated hydrocarbon, such as C3 to C7 alkane or alkene is oxidized ("ammoxidation") in the presence of oxygen, ammonia and the catalyst of the invention to the corresponding nitril. Acrylonitrile or methacrylonitrile can be produced in this manner from propane, propylene, isobutane or isobutene, respectively. This embodiment is conducted under conditions usual in the art involving for instance a reaction temperature of 350 to 500°C, e.g. 400 to 500°C and atmospheric pressure up to 3 atm. As oxygen source air is usually employed.

### EXAMPLES

### X-ray fluorescence measurements (XRF)

XRF measurements were carried out on a Sequenz-Röntgenfluoreszenz-Spektrometersystem S4 PIONEER available from Bruker AXS GmbH, Germany. To further increase the preciseness of the method, standard samples can be prepared that are adapted to the sample to be analyzed such as MoO₃, WO₃, V₂O₅, BiOC1, phosphates such as Zr₃(PO₄)₄ or Ag₃PO₄ and Tea₂O₅ or Nb₂O₅. If required, other suitable standards can be used as known in the art.

**XRD measurements** were performed with a STOE STADI-P transmission diffractometer equipped with a focusing primary Ge (111) monochromator and a position sensitive detector, using Cu-Kα₁ radiation (λ = 1.54 Å). For data analysis, the program TOPAS (v.2.1, Bruker AXS) was used to fit the diffraction patterns of the activated materials.

### Materials

Diluted and undiluted Mo-V-P-Nb mixed metal oxides were prepared by hydrothermal synthesis in a high-throughput (HT) autoclave system. The HT autoclave system is composed of 12 parallel reactors with volume of 140 ml each. The hydrothermal synthesis was carried out at 448 K (175°C) applying reaction times between 10 minutes and 24 hours. SiO₂ (Aerosil 300, Degussa) was used as diluent. All chemicals were purchased from Aldrich and used without further purification.

### Example 1: Mo-V-P-Nb-O mixed oxide diluted with SiO₂ (1/6 hour hydrothermal synthesis)

12-Molybdophosphoric acid, H₃(PMo₁₂O₄₀) ·nH₂O, was synthesized by dissolving 103.64 g MoO₃ using 4 ml phosphoric acid (85 %, ρ = 1.7 kg/1) in bidistilled H₂O and filling up with bidistilled H₂O to a total volume of 1 1. Thereafter, 38.8 ml of the synthesized molybdophosphoric acid solution and another 0.35 ml H₃PO₄ were mixed and further diluted with bidistilled water to a total volume of approximately 45 ml. Then, the solution was heated to 353 K (80°C). Separately, 0.5 g vanadium oxide (V₂O₅) was dispersed in 22.5 ml bidistilled water at 353 K (80°C). The latter mixture was added to the first solution and stirred for 5 minutes. Finally, 0.94 g of ammonium niobium oxalate ((NH₄) [NbO(C₂O₄)₂(H₂O)₂] ·3H₂O was dissolved in 22.5 ml bidistilled water at 353 K (80°C) and added to the previous mixture. The resulting molar ratio of the active metal oxides corresponds to the stoichiometry of Mo₁V_{0.3}P_{0.27}Nb_{0.125}.

The slurry was stirred for 10 minutes at 353 K (80°C) and then SiO₂ (Aerosil 300) was added as diluent in a mass ratio of 1:1 with respect to the weight of the mixture of the stoichiometric oxides.
Subsequently, the slurry was introduced into the autoclave. Residual air was replaced by bubbling nitrogen for 5 minutes through the suspension. After hydrothermal synthesis over 1/6 hour, the resulting gel was filtered, washed and dried at 353 K (80°C) for 16 hours, resulting in the precursor material.

Starting from the precursor, the final catalyst was obtained by heat treatment in inert gas with a flow of 50 ml/min for 2 h at 923 K (650°C), heating rate 15 K/min, with preceding calcination in air for 1 h at 598 K (325°C), heating rate 10 K/min.

The evaluation of the catalyst material is found in example 4.

### Example 2: Mo-V-P-Nb-O mixed oxide diluted with SiO₂ (1/2 hour hydrothermal synthesis)

Example 1 was repeated with the sole difference that hydrothermal synthesis was conducted over ½ hour. The evaluation of the catalyst material is found in example 4.

### Example 3: Mo-V-P-Nb-O mixed oxide diluted with SiO₂ (24 hours hydrothermal synthesis)

Example 1 was repeated with the sole difference that hydrothermal synthesis was conducted over 24 hours. The evaluation of the catalyst material is found in example 4.

### Example 4: Direct oxidation of propane, to acrylic acid

Selective oxidation of propane to acrylic acid was carried out in a setup with twelve parallel fixed bed quartz reactors (i.d., 4 mm; length, 225 mm), working at atmospheric pressure. Catalyst samples (sieve fraction: 0.24 to 0.45 mm particle size) of examples 1 to 3 were introduced into each reactor tube. The feed flow rate was fixed at a gas hourly space velocity (GHSV) of 1200 h⁻¹ (at STP) with standard catalytic bed volume of 0.5 ml. In the feed, a propane/oxygen/nitrogen/steam ratio as shown in table 6 was applied. The reaction was carried out at 673 K (400°C). The products were analyzed by gas chromatography. Inorganic gases and C₁-C₃ hydrocarbons were analyzed with a TCD detector using a molecular sieve column and a Porapak Q column, respectively, for separation. Oxygenated products were detected applying a HP-FFAP column and a flame ionization detector.

The results are shown in the following table 1. It is noted that all catalysts showed an excellent selectivity for the reaction to acrylic acid.

**Table 1**

| Catalytic Performance of Mo-V-P-Nb Oxide | | | | | | | | | |
|---|---|---|---|---|---|---|---|---|---|
| **Example** | **X** | **Diluent** | **HTS* [h]** | **W/F**** | **C_{3/}O_{2/}N_{2/}H₂O [Vol.-%)** | **X_{C3H8} [%]** | **S_{AA} [%]** | **Rate [10⁻³ mmol·g_{Am}⁻¹·h⁻¹]***** | |
| | | | | | | | | **C₃H₈ consumption** | **Formation of acrylic acid** |
| 1 | P | SiO₂ | 1/6 | 560 | 3.33/6.67/60/30 | 5 | 85 | 179 | 152 |
| 2 | P | SiO₂ | 1/2 | 704 | 2.65/19.76/47.78/ 29.81 | 4 | 99 | 114 | 113 |
| 3 | P | SiO₂ | 24 | 560 | 3.33/6.67/60/30 | 5 | 82 | 179 | 146 |

| | | | | | | | | | |
|---|---|---|---|---|---|---|---|---|---|
| * Hydrothermal Synthesis Time ** contact time (g_{cat}·h·mol_{C3H8}⁻¹) T = 673 K; GHSV = 1200 h⁻¹ (STP: T = 273 K, p = 1 atm) *** AM: active mixed oxide | | | | | | | | | |

## Claims

1. Mixed oxide catalyst material which is free of tellurium (Te) and antimony (Sb) and comprises the following elements:
(i) molybdenum (Mo),
(ii) vanadium (V),
(iii) at least one of niobium (Nb), tantalum (Ta) and tungsten (W), and
(iv) phosphorous (P).

2. Mixed oxide catalyst material according to claim 1 wherein the molar ratio P/Mo is at least 0.05/1, preferably at least 0.1/1.

3. Mixed oxide catalyst material according to claim 1 wherein the catalyst material has the general formula (I) :
MO₁VₐX_{b}Q_{c}Z_{d}Oₑ (I)
wherein
X is P, which may be replaced in part by bismuth (Bi),
Q is at least one of Nb, Ta and W,
Z is at least one element selected from Na, K, Si, Ru, Mn, Sc, Ti, Cr, Fe, Co, Ni, Cu, Zn, Ga, Y, Zr, Rh, Pd, In, Sb, Ce, Pr, Nd, Sm, Tb, Re, Ir, Pt, Au and Pb, and a = 0.15-0.50, b = 0.10-0.40, c = 0.05-0.20,
d ≤ 0.05 and e is a number depending on the relative amount and valence of the elements different from oxygen in formula (1).

4. Mixed oxide catalyst material of claim 2, wherein in formula (I)
a = 0.25-0.35, b = 0.20-0.30, c = 0.10-0.15, and d = 0.

5. Mixed oxide catalyst material according to claim 2 wherein in formula (I) Z is at least one of the noble metals Rh, Pd, Ir, Pt and Au, a = 0.25-0.35, b = 0.20-0.30, c = 0.10-0.15, and d = 0.0001 to 0.05.

6. Mixed oxide catalyst material according to any of claims 1 to 5 further containing a diluent.

7. Mixed oxide catalyst material according to claim 6 wherein the diluent is selected from titania (TiO₂), carbon, chromium oxide (Cr₂O₃), alumina, zirconium oxide (zirconia), cerium oxide (CeO₂), SiC and silica.

8. Mixed oxide catalyst material according to claim 7 wherein the diluent is silica.

9. A method for preparing a metal oxide catalyst material as defined in any of claim 1 to 8, said method comprising the steps of
(A) subjecting a catalyst precursor mixture which comprises (i) molybdenum (Mo), (ii) vanadium (V), (iii) at least one of niobium (Nb), tantalum (Ta) and tungsten (W), and (iv) phosphorous (P), and may optionally contain a diluent, to a calcination treatment in an oxygen-containing atmosphere at a temperature of 150 to 400°C, preferably 200 to 350°C to prepare a calcined catalyst material, and
(B) subjecting the catalyst material obtained in step (A) to an activation treatment in an inert gas atmosphere at a temperature of 350 to 700°C, preferably 580 to 670°C.

10. Method according to claim 9 wherein said catalyst precursor mixture is prepared in a method comprising the steps of
(a) dissolving or dispersing starting materials containing at least the catalyst elements (i) to (iv) defined in claim 9, if required under heating,
(b) combining, optionally under heating, the resulting solution(s) and/or dispersion(s) to an aqueous mixture containing the catalyst metals in the desired ratio for the final catalyst material,
(c) heating the aqueous mixture at elevated pressure,
(d) collecting and drying the solid material formed in step (c).

11. Catalyst comprising the catalyst material defined in any of claims 1 to 8 and optionally a carrier.

12. Use of the catalyst of claim 11 as catalyst in oxidation reactions of hydrocarbons or partially oxidized hydrocarbons.

13. Use of claim 12, wherein the hydrocarbons or partially oxidized hydrocarbons are selected from propane, butane, propene, butene and (meth)acrolein and mixtures of two or more of thereof.

14. Use of claim 12 or 13, wherein the oxidized product of the oxidation reaction is (i) at least one of acrylic acid and acrolein or (ii) methacrylic acid.

15. Method for oxidizing propane to acrylic acid in the vapour phase in the presence of oxygen and a catalyst as defined in claim 11.
